# FASCICULE DE BREVET EUROPEEN

(11) **EP 2 355 750 B1**
(45) Date de publication et mention de la délivrance du brevet: **01.03.2017**
(21) Numéro de dépôt: 09809350.3
(22) Date de dépôt: 28.08.2009
(51) Int. Cl.: A61F 2/34, A61F 2/40

(54) **COTYLE PROTHÉTIQUE ARTICULAIRE**
GELENKIGE ACETABULUMPROTHESE
ARTICULAR PROSTHETIC ACETABULUM

(30) Priorité: 01.09.2008 FR 0855839
(43) Date de publication de la demande: 17.08.2011
(73) Titulaire: Pichon, Denis, 35510 Cesson-Sevigne (FR)
(72) Inventeur: Pichon, Denis, 35510 Cesson-Sevigne (FR)
(74) Mandataire: Regimbeau
(86) Numéro de dépôt international: PCT/EP2009/061172
(87) Numéro de publication internationale: WO 2010/023296

(56) Documents cités:
- EP-A- 0 694 294
- WO-A-00/64383
- DE-A1- 19 654 106
- DE-A1- 19 926 923
- US-A1- 2007 179 629

## Description

La présente invention a pour objet un cotyle prothétique articulaire.

Elle trouve notamment application dans l'arthroplastie totale de la hanche, dans laquelle l'implant prothétique comporte une liaison de type « conicité à pente réduite » utilisée pour la connexion de deux éléments constitutifs du cotyle prothétique de la hanche.

Une prothèse totale de l'articulation de la hanche, qui est mise en place dans une telle opération, comprend essentiellement deux parties, l'une « mâle » destinée à se substituer à la tête du fémur, l'autre « femelle » destinée à être placée dans la cavité cotyloïdienne de l'os iliaque du bassin, dans laquelle était insérée la tête du fémur.

La partie mâle comporte une tige apte à être implantée dans le canal médullaire de l'extrémité proximale du fémur, ainsi qu'une tête quasi sphérique, tandis que la partie femelle comporte un cotyle apte à être inséré et fixé, potentiellement à l'aide de plusieurs vis, dans la cavité naturelle de l'os du bassin.

Ce cotyle prothétique a la configuration générale d'une cupule dont la paroi externe convexe est adaptée à celle de la cavité naturelle de l'acetabulum, tandis que sa paroi interne concave est garnie d'un insert -ou coque- pourvue d'une cavité de forme sensiblement hémisphérique, complémentaire de la tête de la partie mâle.

Après insertion et ancrage de cette dernière dans le cotyle prothétique, on obtient une articulation à rotule, stable et durable, dont les caractéristiques fonctionnelles sont proches de celles de l'articulation naturelle de la hanche.

Il convient de noter que les couples de matériaux utilisés au niveau de l'interface de frottement tête de fémur/coque ou insert sont généralement les suivants : métal/métal, métal/polyéthylène, céramique/céramique, céramique/ polyéthylène, composite/métal, composite/céramique, céramique/composite, ou tous matériaux présentant les caractéristiques tribologiques adéquates.

La cupule externe est généralement en métal, par exemple en alliage de chrome/cobalt, de titane, ou autre matériau adéquat.

Dans le domaine des prothèses orthopédiques, il est relativement courant d'assurer la liaison entre deux pièces par un emmanchement de cônes de faible pente, dit « cônes morses ».

Les deux pièces à assembler, l'une mâle, l'autre femelle, possèdent des parois emboîtables à formes tronconiques complémentaires d'angle au sommet relativement faible, à titre indicatif compris entre 1° et 20° environ (soit un demi-angle au sommet compris entre 0,5° et 10° environ).

Ce mode de liaison est à la fois simple à réaliser et très efficace, assurant un très bon centrage et une solidarisation sûre des deux pièces l'une à l'autre, ceci aussi bien en rotation qu'en translation.

Une telle liaison peut être prévue aussi bien du côté fémur, pour assurer la fixation de la tête sur le col de la tige médullaire (voir par exemple le document FR 2 678 162), que du côté bassin, pour assurer la fixation de la coque dans la cupule de cotyle (voir par exemple le document US 5 919 236).

Une liaison du type cône morse est réellement efficace lorsque chacune des deux pièces à assembler, en l'occurrence la cupule et la coque, est en matériau dur.

La cupule étant généralement métallique, une telle liaison convient bien pour l'insertion dans cette cupule d'une coque également en métal ou en céramique.

L'invention a pour objet un cotyle dont l'assemblage se fait par effet de cône morse, la coque étant en matériau dur, et de type métal ou céramique.

Un problème traditionnellement rencontré avec ce genre d'assemblage se pose lorsqu'il s'agit d'extraire l'ensemble du cotyle, ou seulement la coque, afin de le (la) remplacer.

En effet, la liaison par cône morse est très efficace, extrêmement difficilement démontable en per opératoire en cas de nécessité d'ablation de l'implant.

Or, pour accéder aux vis d'ancrage de la cupule, dont la tête est située derrière la face externe de la coque, il est nécessaire d'enlever préalablement la coque.

Des solutions empiriques et non satisfaisantes doivent alors être mises en oeuvre par le chirurgien, qui impliquent notamment la fracturation de la coque en céramique (ce qui génère la production de particules indésirables néfastes au fonctionnement et à la longévité du nouvel implant qui sera mis en place), voire le sectionnement des vis par l'extérieur du cotyle, entre la cupule et la cavité cotyloïdienne naturelle du patient (ce qui peut causer des lésions et des traumatismes importants de l'os du bassin).

Dans le document US 5 879 397, la paroi interne tronconique de l'embouchure de la cupule (dans la zone de liaison par cône morse) présente une paire de rainures diamétralement opposées- destinée à permettre l'insertion d'un outil spécial en vue du démontage. Cet outil a la configuration d'une canne de golf, comprenant une tige dont la partie d'extrémité, qui correspond au « fer » de la canne de golf, peut être insérée sous la coque et, par rotation de la tige, provoquer par effet de coin ou de bras de levier l'extraction de la coque hors de la cupule.

En pratique cette solution est très délicate à mettre en oeuvre.

Lorsque l'effort de torsion développé par le chirurgien n'est pas suffisant pour obtenir la rupture d'adhérence nécessaire, celui-ci est incité à effectuer complémentairement à une traction axiale sur les outils, laquelle se répercute via la cupule aux vis d'ancrage, induisant un risque de lésions du tissu osseux environnant.

Dans le document DE 19926923, il est prévu une bague intermédiaire destinée à être insérée entre la coque et la cupule et un espace est ménagé à certains endroits entre ladite bague et ladite cupule pour pouvoir y insérer un outil de démontage et atteindre ainsi la face inférieure de ladite bague.

Toutefois, ce genre de dispositif nécessite l'emploi d'un outil additionnel de démontage que le chirurgien n'a pas forcément à sa disposition, notamment lorsqu'il doit opérer en urgence.

On connaît enfin d'après le document EP 0 694 294 un cotyle prothétique dans lequel la bague est solidarisée à la fois à la cupule et à la coque par une fixation de type « cône Morse ».

Le démontage de la bague et de la cupule est donc difficile.

La présente invention a pour objectif de résoudre ces difficultés, en proposant un cotyle du genre indiqué plus haut, dans lequel l'insertion de la coque à l'intérieur de la cupule, après ancrage de cette dernière au moyen de vis dans la cavité cotyloïdienne de l'os iliaque du patient, se fait de manière habituelle pour le chirurgien, par emmanchement axial et assemblage du type cône morse, cette coque étant néanmoins très aisément extractible au besoin, en vue notamment de donner accès aux vis d'ancrage pour les dévisser et retirer la cupule.

L'objet de l'invention est donc un cotyle prothétique, notamment pour l'articulation de la hanche ou de l'épaule, comportant :
- une cupule en matériau dur et biocompatible, ayant une forme générale approximativement hémisphérique, à symétrie de révolution autour d'un axe **(X-X')** -dit central- et présentant une face externe convexe adaptée pour être insérée et fixée dans la cavité cotyloïdienne de l'os;
- une coque en matériau implantable, notamment en métal, en céramique ou en matériau composite biocompatible, apte à se loger à l'intérieur de ladite cupule, cette coque présentant une cavité interne sensiblement hémisphérique, destinée à recevoir la tête d'une prothèse articulaire, et possédant une embouchure dont la paroi externe présente une forme tronconique, de faible conicité, s'évasant vers l'extérieur ;
- une bague apte à être fixée de manière démontable à l'embouchure de ladite cupule et présentant une paroi interne également tronconique, dont la forme est complémentaire de celle de la paroi externe de l'embouchure de la coque, de sorte qu'il est possible d'y emmancher ladite coque en assurant un assemblage du type cône morse entre la bague et la coque.

Conformément à l'invention, ladite cupule présente une cavité interne bordée par une embouchure, tandis que ladite bague possède une portion à paroi externe dont la surface présente une forme complémentaire de celle de la surface de ladite embouchure, de façon à pouvoir être insérée axialement à l'intérieur de ladite embouchure et à être démontable par rapport à ladite cupule et par le fait que la bague et ladite cupule peuvent être démontées par une action de rotation de l'une des pièces constituant ledit cotyle autour d'un axe longitudinal parallèle audit axe central ou incliné.

De préférence, la bague peut être démontée par rapport à ladite cupule par une action de vissage et/ou de dévissage.

Grâce à cet arrangement, il est aisé de démonter « en bloc » le sous-ensemble bague/coque, sans avoir à rompre l'adhérence de l'emboîtement à cône morse qui maintient ces deux pièces solidaires l'une de l'autre.

Par ailleurs, selon un certain nombre de caractéristiques additionnelles possibles de l'invention :
- ladite cupule présente une cavité interne qui comprend une zone de fond concave bordée par une embouchure dont au moins une partie est cylindrique, tandis que ladite bague possède une paroi externe de forme complémentaire dont au moins une partie est également cylindrique, l'une des ces deux parties cylindriques portant un taraudage et l'autre un filetage, de sorte que ladite bague et ladite cupule peuvent être démontées par une action de vissage ou de dévissage ;
- ladite bague est traversée par plusieurs trous taraudés d'axe longitudinal parallèle audit axe central **(X-X')** ou inclinés, aptes à recevoir des tiges filetées en vue du démontage du sous-ensemble composé de la bague et de la coque, par rapport à ladite cupule, ce démontage étant effectué par une extraction résultant de la pression exercée par l'extrémité de ces tiges filetées logées dans la bague contre la cupule lorsqu'elles sont soumises à une rotation ;
- ladite bague possède une embase annulaire externe, apte à prendre appui contre le chant de la cupule lorsque la bague est fixée à cette dernière ;
- lesdits trous taraudés sont ménagés dans ladite embase annulaire externe ;
- ladite bague est percée de plusieurs orifices pour le passage de vis de fixation destinées à être vissées dans des trous taraudés, d'axe longitudinal parallèle audit axe central, ménagés dans la paroi de la cupule ;
- ladite cupule présente une cavité interne qui comprend une zone de fond concave bordée par une embouchure cylindrique tandis que ladite bague possède une portion à paroi externe également cylindrique et de même diamètre, de sorte qu'elle peut être insérée axialement et pratiquement sans jeu à l'intérieur de ladite embouchure ;
- la paroi interne de l'embouchure de la cupule a une forme étagée, composée de l'intérieur vers l'extérieur de la cupule, de deux zones cylindriques de diamètres différents, celle de plus grand diamètre étant située du côté de l'ouverture de la cupule, ces deux zones étant reliées par une zone annulaire plane perpendiculaire audit axe central, et ladite portion à paroi externe cylindrique de la bague possède une forme étagée complémentaire, dont le décrochement forme un épaulement annulaire apte à prendre appui contre ladite zone annulaire plane lorsque la bague est fixée à la cupule ;
- ladite cupule présente une cavité interne qui comprend une zone de fond concave bordée par une embouchure dont au moins une partie de la paroi interne présente une forme tronconique, de grande conicité, s'évasant vers l'extérieur, tandis que ladite bague possède une portion à paroi externe de forme complémentaire, de sorte qu'elle peut être insérée axialement et pratiquement sans jeu à l'intérieur de ladite embouchure et être démontable par rapport à ladite cupule.

Un tel cotyle trouve notamment application pour l'articulation de la hanche, sa cupule étant alors adaptée pour être insérée et fixée dans la cavité cotyloïdienne de l'os du bassin, tandis que sa coque reçoit la tête d'une prothèse fémorale.

Il peut également s'appliquer à l'articulation de l'épaule, sa cupule étant adaptée dans ce cas pour être insérée et fixée dans la glène de l'omoplate, tandis que sa coque reçoit la tête d'une prothèse humérale.

D'autres caractéristiques et avantages de la présente invention apparaîtront à la lumière de la description qui va maintenant en être faite, en référence aux dessins annexés.

Ces dessins représentent, à titre d'exemple non limitatif, un mode d'exécution préféré du cotyle prothétique faisant l'objet de l'invention, ainsi qu'une variante de celui-ci.
La figure 1 est une vue éclatée et en perspective montrant, avant assemblage, les trois principaux éléments constitutifs de ce cotyle, ainsi que les vis d'assemblage de la bague avec la cupule.
La figure 2 est une vue éclatée similaire, mais en coupe axiale.
La figure 3 est une vue similaire à celle de la figure 2, après assemblage des éléments composant le cotyle prothétique.
La figure 4 est une vue analogue à celle de la figure 4, montrant une variante du cotyle.

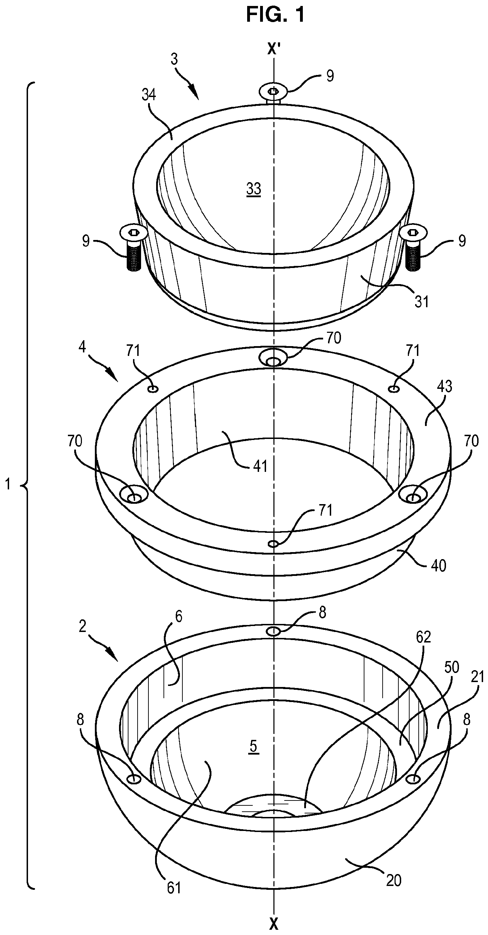

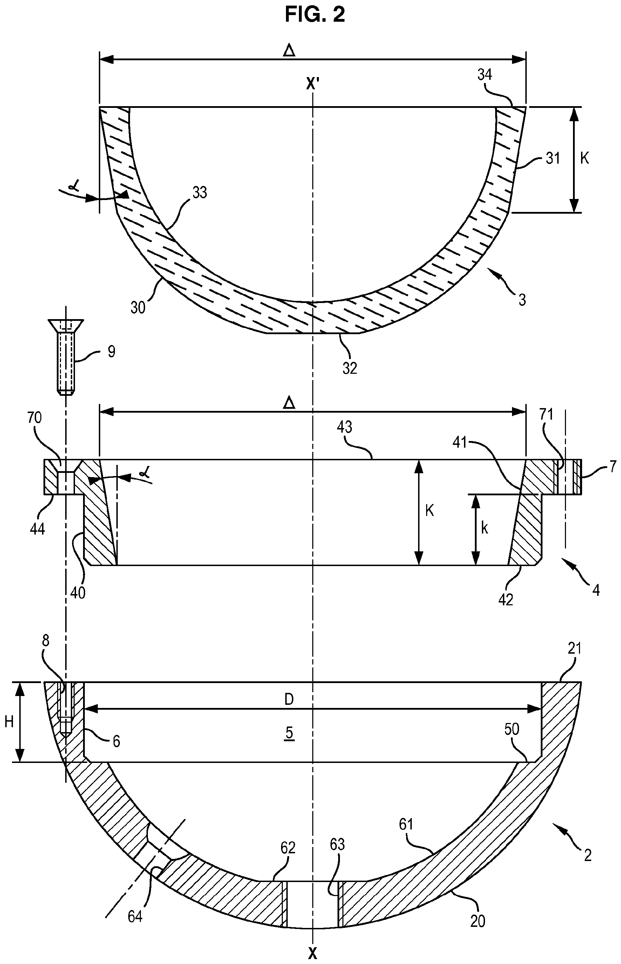

Comme cela est notamment visible sur les figures 1 et 2, le cotyle prothétique illustré, qui porte la référence 1, est composé de trois pièces principales, à savoir une cupule 2, une coque 3 et une bague 4, et également d'au moins une vis d'assemblage 9. Sur ces figures, trois vis 9 sont représentées.

La cupule 2 est rigide, de préférence en métal ou en un autre matériau dur et biocompatible.

Il s'agit d'une pièce de révolution, d'axe de symétrie **X-X'**, que l'on appellera « axe central ».

Sa face externe 20 est convexe, par exemple à surface approximativement hémisphérique, adaptée pour se loger dans la cavité cotyloïdienne de l'os, par exemple l'os du bassin, et y être fixée.

De façon connue, cette fixation est réalisée au moyen de plusieurs vis d'ancrage qui traversent des orifices adéquats percés radialement dans la paroi de la cupule, ces orifices pouvant être chanfreinés pour noyer la tête fraisée de la vis qui les traverse.

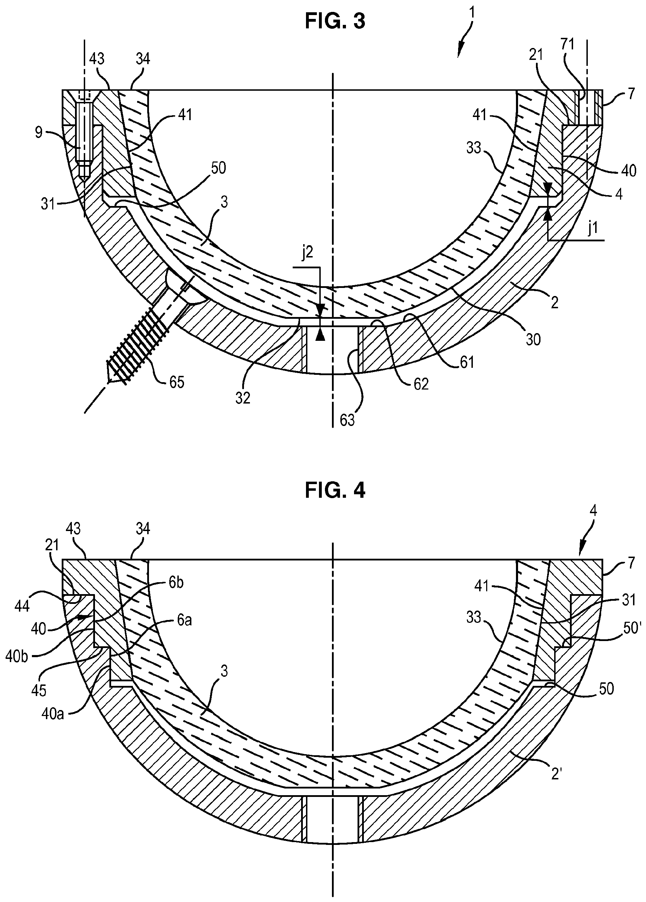

L'un de ces orifices, non représentés sur les figures 1 et 4, est représenté sur les figures 2 et 3 ; il porte la référence 64 tandis que la vis d'ancrage, qui le traverse, visible sur la figure 3, porte la référence 65.

Comme on le voit sur la figure 2, la cupule 2 présente une cavité interne 5, composée d'une paroi de fond 61-62 et d'une paroi d'embouchure 6.

Selon un premier mode de réalisation, la paroi 6 est cylindrique, de diamètre **D** et de hauteur **H** (dimension suivant **X-X'**).

La paroi de fond présente une zone centrale plane 62, perpendiculaire à l'axe central **X-X'**, qui est bordée par une zone concave 61, à surface sphérique ou approximativement sphérique.

La paroi de fond est traversée par un trou axial taraudé 63, dont la fonction est de permettre la manipulation et le positionnement de la cupule 2 dans l'os du bassin à l'aide d'une tige d'ancillaire à extrémité filetée, ceci selon une disposition connue en soi.

La référence 21 désigne le chant extérieur de la cupule 2, tandis que la référence 50 désigne la zone annulaire assurant la transition entre la portion cylindrique 6 et l'entrée de la paroi de fond 61, dont le diamètre est inférieur à **D.**

Cette zone 50 est plane, et s'inscrit dans un plan transversal, perpendiculaire à **X-X'**.

La coque 3 est en matière dure et rigide, par exemple en céramique.

Elle présente une cavité interne 33 sensiblement hémisphérique, d'axe de symétrie **X-X'**, adaptée pour recevoir la tête quasi-sphérique de la prothèse fémorale ou humérale (non représentée).

Sa face externe comprend une portion de fond convexe 30 à fond plat 32.

Cette portion de fond se prolonge vers l'extérieur par une portion tronconique et lisse 31, convergeant en direction de la cupule 2, de hauteur **K.**

Avantageusement sa conicité est de l'ordre de 1° à 20°, ce qui correspond à un demi-angle au sommet **α** de valeur comprise entre 0,5° et 10°, ou voisine de cette valeur.

La bague 4 est en matériau dur et rigide, avantageusement en métal.

Elle a la configuration générale d'un tronçon de tube d'axe **X-X',** dont la paroi interne 41 est également lisse et conique, convergeant du côté du fond de cupule 5, et dont la paroi externe 40 est cylindrique.

La conicité de la paroi 41 est la même que celle de la paroi 31 (demi-angle au sommet **α).**

Le diamètre d'entrée de la paroi 41 est égal au diamètre externe de la paroi 31 au niveau du chant 34, ce diamètre commun étant désigné **Δ** sur la figure 2. Cette paroi 41 a la même hauteur **K** que celle de la portion de paroi 31 de la coque 3.

La paroi externe de la bague 4 est étagée, comprenant, tournée vers le fond de cupule, une portion principale cylindrique 40 dont le diamètre correspond au diamètre **D** de la paroi interne 6 de la cupule, et - du côté extérieur- une embase (collerette) 7 également cylindrique, mais de plus grand diamètre, égal au diamètre externe du chant 21 de la cupule 2.

Le chant extérieur de la bague 4 porte la référence 43.

La face intérieure (côté fond de cupule) de l'embase 7, de forme annulaire, porte la référence 44.

La portion 40 possède une hauteur **k** qui est légèrement inférieure à la hauteur **H** de la cupule. Cette portion est destinée à être emmanchée axialement sans jeu notable à l'intérieur de la cupule, jusqu'à ce que l'embase vienne en appui, par sa face 44, contre le chant 21 de la cupule. Un certain jeu, référencé **j1** sur la figure 3 subsiste alors entre l'extrémité intérieure de la bague 4 et la zone annulaire 50 de la cupule.

L'embase est traversée de deux séries d'orifices d'axe parallèle à **X-X',** à savoir une série de trous cylindriques 70 à entrée chanfreinée (tronconique) et une série de trous taraudés 71.

Dans le mode de réalisation illustré, chacune de ces séries est composée de trois trous disposés à 120° par rapport à l'axe central **X-X'**, les trous 70 étant décalés de 60° par rapport aux trous 71.

On obtient ainsi une répartition angulaire régulière des six orifices.

La paroi d'embouchure de la cupule 2, quant à elle, est percée de trois trous taraudés 8, également répartis à 120° et d'axe parallèle à **X-X**'.

Les orifices 70 et les trous 8 sont ainsi positionnés et dimensionnés qu'ils peuvent être mis en coïncidence si l'embase est correctement orientée en rotation autour de l'axe central **X-X**' et que la portion principale de la bague 4 est complètement emmanchée dans l'embouchure de cupule.

Il est alors possible de solidariser la bague et la cupule au moyen de trois vis de serrage 9 à tête fraisée, qui sont vissées à fond dans les trous 8 et dont la tête se trouve noyée dans le chanfrein d'entrée des trous 70.

La face annulaire 44 de l'embase 7 se trouve alors intimement appliquée contre le chant 21 de la cupule.

Il est à noter que les zones du chant 21 situées en regard des trous taraudés 71 sont pleines (non percées).

Selon une variante non représentée sur les figures, l'embase annulaire 7 pourrait être supprimée et chaque paire de trous 8 et 70 coaxiaux superposés pourrait être remplacée par un unique trou d'axe parallèle à l'axe X-X' ménagé à l'intersection entre les plans de contact entre la cupule 2 et la bague 4.

Nous allons maintenant expliquer comment un tel cotyle est implanté.

Le chirurgien commence par insérer et positionner correctement la cupule 2 dans l'os du bassin, à l'aide d'une tige de manoeuvre vissée dans le trou taraudé de fond de cupule 63, puis l'y fixe au moyen de plusieurs vis d'ancrage 65 traversant les trous 64 de la cupule.

Il insère ensuite axialement la coque 3 à l'intérieur de la bague 4, de manière à obtenir une liaison à cône morse entre les parois coniques complémentaires « mâle » 31 et femelle « 41 » et vérifie que ces deux pièces sont correctement positionnées l'une par rapport à l'autre, notamment en ce que les chants 34 de la coque et 43 de la bague sont rigoureusement dans le même plan transversal (perpendiculaire à X-X'). Si tel n'est pas le cas, il peut éventuellement les démonter par pression sur le fond de la coque 3, puis les réassembler correctement et ce, très aisément et de façon fiable puisque ceci s'effectue à l'écart du patient sur lequel la prothèse doit être implantée.

Cet assemblage se fait de manière classique, par exemple à l'aide d'un outil impacteur.

Le chirurgien solidarise ensuite le sous-ensemble coque 3 / bague avec la cupule 2 à l'aide des vis 9, de la manière indiquée plus haut.

Les formes et dimensions respectives des différents éléments (cupule, bague et coque) sont choisies pour qu'après assemblage, illustré sur la figure 3, non seulement les chants 34 de la coque et 43 de la bague s'inscrivent sensiblement dans le même plan transversal (perpendiculaire à **X**-**X**'), mais qu'il existe un certain jeu entre la paroi de fond externe convexe 30-32 de la coque 3 et la paroi interne concave 61-62 de la cupule 2.

Le jeu entre les faces en regard 32 et 62 est désigné j2 sur la figure 3.

On notera que pour le chirurgien, la technique d'implantation à mettre en oeuvre est plus simple que pour l'implantation d'un cotyle classique, composé d'une simple cupule métallique recevant directement, par emmanchement et liaison de type cône morse, une coque en céramique ou en métal.

La différence est également manifeste pour ce qui est du retrait ultérieur de la coque.

Un tel retrait peut être voulu, soit pour remplacer la coque usagée, et lui substituer une nouvelle coque, soit pour accéder aux vis d'ancrage 65, dont la tête se trouve en dessous de la coque, afin de retirer la cupule dans le but de remplacer l'ensemble du cotyle.

La méthode pour retirer la coque est ici très simple et aisée à mettre en oeuvre.

Il suffit pour cela, dans un premier temps de dévisser et de retirer les vis de serrage 9 et, dans un second temps, d'engager des tiges à extrémité filetées dans les trous taraudés 71 et de les faire tourner sur elles-mêmes dans leur sens de leur enfoncement dans ces trous (par vissage ou dévissage selon le sens du pas de vis).

Lorsque l'extrémité d'une tige vient porter contre le chant 21 de la cupule, et dès lors que sa mise en rotation se poursuit, elle développe un effort axial qui entraîne le « décollement » des faces en appui 44 et 21. Alternativement, un instrument adéquat peut être utilisé pour générer un arrachement de ladite bague au moyen des vis saillantes, les vis étant elles-mêmes placées dans les trous taraudés n'ayant pas de vis-à-vis en creux au niveau de la cupule métallique.

La bague 4 et la coque 3 dont elle est restée solidaire peuvent être alors facilement retirées en bloc hors de la cupule.

Les vis d'ancrage 65 de la cupule 2 deviennent ainsi accessibles.

Les tiges filetées coopérant avec les trous taraudés 71 peuvent être simplement des tiges de vis.

Les vis 9 pourraient également être sécables ou remplacées par des éléments de liaison sécables. Dans ce cas, le démontage s'effectue par un mouvement de rotation relatif de la bague 4 par rapport à la cupule 2, autour de l'axe central **X-X'.**

On notera que durant cette opération le chirurgien n'a pas été obligé de désassembler l'emboîtement à cône morse qui solidarise la coque et la bague ; il a pu les extraire en bloc, avec un effort minime.

Le cas échéant le désassemblage peut se faire ultérieurement en dehors du champ opératoire, et au moyen d'outils adaptés.

La variante de cotyle représentée sur la figure 4 se distingue du précédent uniquement par la forme de la paroi externe de la portion cylindrique 40 de la bague 4.

Cette paroi a une section de forme étagée, comprenant deux parties cylindriques de diamètres différents 40a, 40b ; la partie 40a tournée vers le fond de la cupule a un plus petit diamètre que celle située du côté de l'embase 7 ; ces deux parties sont reliées par un épaulement annulaire 45 s'inscrivant dans un plan transversal.

La paroi interne de l'embouchure 6 de la cupule possède une section de forme étagée complémentaire, comprenant une partie 6a dans laquelle s'emmanche la partie 40a et une partie 6b dans laquelle s'emmanche la partie 40b ; les parties 6a et 6b se raccordent par une zone annulaire 50' s'inscrivant dans un plan transversal, parallèlement à la zone annulaire 50.

Selon cette variante, l'appui de la bague 4 dans l'embouchure de la cupule se fait non seulement au niveau de l'embase 7 (entre les faces 44 et 21), mais aussi au niveau de l'épaulement 45 et de la zone 50', ce qui améliore le centrage et l'assise de la bague dans la cupule.

Les trous 70 et 71 n'ont pas été représentés sur la figure 4 à des fins de simplification. Ils pourraient toutefois être présents et le montage et le démontage des pièces s'effectuent alors comme décrit précédemment.

Dans le mode de réalisation qui a été illustré sur les dessins, il est fait usage d'une série de trois orifices 70 destinés à recevoir les vis de serrage 9 et de trois trous taraudés 8. Des nombres différents d'orifices et/ou de trous taraudés peuvent évidemment être prévus, par exemple quatre orifices 70 disposés à 90° et une paire de trous taraudés 8 diamétralement opposés, avec une orientation à 45° par rapport aux orifices 70.

En outre, la solidarisation initiale entre la cupule et la bague n'est pas nécessairement faite au moyen de vis de serrage. L'important est que la bague puisse être aisément démontée in situ, notamment par vissage et/ou dévissage, afin que le sous-ensemble bague/coque soit facilement extractible.

Comme autre solution possible, on peut mentionner par exemple une solidarisation par vissage de la bague 4 dans l'embouchure de la cupule 2, la paroi 40 pouvant être filetée et la paroi 6 taraudée (ou inversement). Dans cette autre variante, les trous 70 et 71 ne sont pas présents.

L'extraction du sous-ensemble bague/coque s'obtient alors par dévissage.

Dans la variante à plusieurs étage de la figure 4 et lorsqu'on utilise par les vis de serrage 9, le filetage et le taraudage seront prévus au choix sur les parties 6a, 40a ou sur les parties 6b, 40b.

Enfin, on notera que l'embouchure 6 et la paroi 40 peuvent présenter des formes complémentaires autres que cylindriques.

Il peut s'agir par exemple d'une forme tronconique, de grande conicité, s'évasant vers l'extérieur. Par "grande conicité", on entend une conicité supérieure à 24°, ce qui correspond à un demi-angle au sommet de valeur supérieure à 12°. Cette forme est très aisément démontable.

Il peut également s'agir d'une forme spécifique définie par le déplacement d'une génératrice le long du contour d'une surface quelconque, parallèlement à l'axe X-X', par exemple la surface d'un polygone ou une surface polylobée.

L'invention peut s'appliquer à d'autres types de prothèse, notamment pour l'articulation de l'épaule.

## Revendications

1. Cotyle prothétique articulaire, notamment pour l'articulation de la hanche ou de l'épaule, comportant :
- une cupule (2) en matériau rigide et biocompatible, ayant une forme générale approximativement hémisphérique, à symétrie de révolution autour d'un axe **(X-X')** -dit central- et présentant une face externe convexe (20) adaptée pour être insérée et fixée dans la cavité cotyloïdienne de l'os;
- une coque (3) en matériau implantable, notamment en métal, en céramique ou en matériau composite biocompatible, apte à se loger à l'intérieur de ladite cupule (2), cette coque (3) présentant une cavité interne (33) sensiblement hémisphérique, destinée à recevoir la tête d'une prothèse articulaire, et possédant une embouchure dont la paroi externe (31) présente une forme tronconique, de faible conicité, s'évasant vers l'extérieur ;
- une bague (4) apte à être fixée de manière démontable à l'embouchure de ladite cupule (2) et présentant une paroi interne (41) également tronconique, dont la forme est complémentaire de celle de la paroi externe (31) de l'embouchure de la coque (3), de sorte qu'il est possible d'y emmancher ladite coque (3) en assurant un assemblage du type cône morse entre la bague (4) et la coque (3),
ladite cupule (2) présentant une cavité interne (5) bordée par une embouchure (6), tandis que ladite bague (4) possède une portion à paroi externe (40) dont la surface présente une forme complémentaire de celle de la surface de ladite embouchure (6), de façon à pouvoir être insérée axialement à l'intérieur de ladite embouchure (6) et être démontable par rapport à ladite cupule (2),
**caractérisé par le fait que** ladite bague (4) et ladite cupule (2) sont solidarisées par des moyens de vissage, ladite bague (4) possédant une embase annulaire externe (7), apte à prendre appui contre le chant (21) de la cupule (2) lorsque la bague (4) est fixée à cette dernière, cette embase annulaire externe (7) étant percée de plusieurs orifices (70) pour le passage de vis de fixation (9) destinées à être vissées dans des trous taraudés (8), d'axe longitudinal parallèle audit axe central **X-X',** ménagés dans la paroi de la cupule (2), ces vis de fixation (9) et ces trous taraudés (8) constituant lesdits moyens de solidarisation par vissage, et **par le fait que** la bague (4) et ladite cupule (2) sont démontables par dévissage desdites vis de fixation (9).

2. Cotyle prothétique selon la revendications 1, **caractérisé par le fait que** ladite cupule (2) présente une cavité interne (5) qui comprend une zone de fond (61, 62) concave bordée par une embouchure cylindrique (6), tandis que ladite bague (4) possède une portion à paroi externe (40) également cylindrique et de même diamètre, de sorte qu'elle peut être insérée axialement et pratiquement sans jeu à l'intérieur de ladite embouchure (6).

3. Cotyle prothétique selon la revendication 2, **caractérisé par le fait que** l'embouchure (6) de la cupule (2) a une forme étagée, composée de l'intérieur vers l'extérieur de la cupule, de deux parties (6a ; 6b) de diamètres différents, celle de plus grand diamètre (6b) étant située du côté de l'ouverture de la cupule, ces deux parties étant reliées par une zone annulaire plane (50') perpendiculaire audit axe central **X-X',** et **par le fait que** ladite portion de paroi externe cylindrique (40) de la bague (4) possède une forme étagée complémentaire, dont le décrochement forme un épaulement (45) apte à prendre appui contre ladite zone annulaire plane (50') lorsque la bague (4) est fixée à la cupule (2).

4. Cotyle prothétique selon la revendication 1, **caractérisé par le fait que** ladite cupule (2) présente une cavité interne (5) qui comprend une zone de fond (61, 62) concave bordée par une embouchure (6) dont au moins une partie de la paroi interne présente une forme tronconique, de grande conicité, s'évasant vers l'extérieur, tandis que ladite bague (4) possède une portion à paroi externe (40) de forme complémentaire, de sorte qu'elle peut être insérée axialement et pratiquement sans jeu à l'intérieur de ladite embouchure (6) et être démontable par rapport à ladite cupule (2).

5. Cotyle prothétique selon la revendication 4, **caractérisé en ce que** la forme tronconique de grande conicité correspond à un demi-angle au sommet supérieur à 12°.

6. Cotyle prothétique selon l'une des revendications précédentes, **caractérisé par le fait que** ladite bague (4) est traversée par plusieurs trous taraudés (71) d'axe longitudinal parallèle audit axe central **(X-X')** ou inclinés, aptes à recevoir des tiges filetées, telles que des tiges de vis, en vue du démontage du sous-ensemble composé de la bague (4) et de la coque (3), par rapport à ladite cupule (2), ce démontage étant effectué par une extraction résultant de la pression exercée par l'extrémité de ces tiges filetées logées dans la bague (4) contre la cupule (2) lorsqu'elles sont soumises à une rotation.

7. Cotyle prothétique selon la revendication 5, **caractérisé par le fait que** lesdits trous taraudés (71) sont ménagés dans ladite embase annulaire externe (7).

## Patentansprüche

1. Gelenkige Acetabulumprothese, insbesondere für das Hüft- oder Schultergelenk, aufweisend:
- einen Becher (2) aus festem und biologisch kompatiblem Material, der allgemein etwa halbkugelförmig ist, mit Drehsymmetrie um eine zentrale Achse (X-X'), und eine konvexe Außenfläche (20) aufweist, die ausgebildet ist, um in die Gelenkpfannenhöhle des Knochens eingesetzt und befestigt zu sein,
- eine Schale (3) aus implantierbarem Material, insbesondere aus Metall, aus Keramik oder aus biologisch kompatiblem Verbundmaterial, die imstande ist, sich in das Innere des Bechers (2) einzufügen, wobei diese Schale (3) eine etwa halbkugelförmige innere Höhle (33) aufweist, die bestimmt ist, den Kopf einer Gelenkprothese aufzunehmen, und eine Mündung besitzt, deren Außenwand (31) eine schwach konische, sich nach außen erweiternde Kegelstumpfform aufweist,
- einen Ring (4), der imstande ist, demontierbar an der Mündung des Bechers (2) befestigt zu sein und eine ebenfalls kegelstumpfförmige Innenwand (41) aufweist, deren Form zu der der Außenwand (31) der Mündung der Schale (3) komplementär ist, so dass es möglich ist, dort die Schale (3) bei Gewährleistung einer Verbindung vom Typ Morsekonus zwischen dem Ring (4) und der Schale (3) einzufügen,
wobei der Becher (2) eine innere Höhle (5) aufweist, die von einer Mündung (6) gesäumt ist, wogegen der Ring (4) einen Außenwandabschnitt (40) besitzt, dessen Oberfläche eine zur Oberfläche der Mündung (6) komplementäre Form aufweist, so dass er axial in das Innere der Mündung (6) einsetzbar ist und in Bezug zu dem Becher (2) demontierbar ist,
**dadurch gekennzeichnet, dass** der Ring (4) und der Becher (2) mit Hilfe von Schraubenmitteln fest verbunden sind, wobei der Ring (4) eine äußere ringförmige Basis (7) besitzt, die imstande ist, sich auf der Kante (21) des Bechers (2) abzustützen, wenn der Ring (4) an diesem befestigt ist, wobei diese äußere ringförmige Basis (7) von mehreren Öffnungen (70) für den Durchgang von Befestigungsschrauben (9) durchbrochen ist, die bestimmt sind, in Gewindelöcher (8) länglicher, zur zentralen Achse X-X' paralleler Achse eingeschraubt zu sein, die in der Wand des Bechers (2) ausgebildet sind, wobei diese Befestigungsschrauben (9) und diese Gewindelöcher (8) die Verbindungsmittel durch Schrauben darstellen, und dadurch, dass der Ring (4) und der Becher (2) durch Lösen der Befestigungsschrauben (9) demontierbar sind.

2. Acetabulumprothese nach Anspruch 1, **dadurch gekennzeichnet, dass** der Becher (2) eine innere Höhle (5) aufweist, die eine konkave Bodenzone (61, 62) umfasst, die von einer zylindrischen Mündung (6) gesäumt ist, wogegen der Ring (4) einen ebenfalls zylindrischen Außenwandabschnitt (40) mit demselben Durchmesser besitzt, damit er axial und praktisch ohne Spiel in das Innere der Mündung (6) einsetzbar ist.

3. Acetabulumprothese nach Anspruch 2, **dadurch gekennzeichnet, dass** die Mündung (6) des Bechers (2) eine stufige Form hat, die vom Inneren zum Äußeren des Bechers aus zwei Teilen (6a; 6b) mit unterschiedlichen Durchmessern besteht, wobei sich der Teil mit größerem Durchmesser (6b) auf der Seite der Öffnung des Bechers befindet, wobei diese zwei Teile durch eine ebene ringförmige Zone (50'), die senkrecht zur zentralen Achse X-X' ist, verbunden sind, und dadurch, dass der zylindrische Außenwandabschnitt (40) des Rings (4) eine komplementäre stufige Form besitzt, dessen Rücksprung einen Absatz (45) bildet, der imstande ist, sich auf der ebenen ringförmigen Zone (50') abzustützen, wenn der Ring (4) am Becher (2) befestigt ist.

4. Acetabulumprothese nach Anspruch 1, **dadurch gekennzeichnet, dass** der Becher (2) eine innere Höhle (5) aufweist, die eine konkave Bodenzone (61, 62) umfasst, die von einer Mündung (6) gesäumt ist, von der mindestens ein Teil der Innenwand eine stark konische, sich nach außen erweiternde Kegelstumpfform aufweist, wogegen der Ring (4) einen komplementär geformten Außenwandabschnitt (40) besitzt, so dass er axial und praktisch ohne Spiel in das Innere der Mündung (6) einsetzbar ist und in Bezug zu dem Becher (2) demontierbar ist.

5. Acetabulumprothese nach Anspruch 4, **dadurch gekennzeichnet, dass** die stark konische Kegelstumpfform einem Halbwinkel an der Spitze von über 12° entspricht.

6. Acetabulumprothese nach einem der vorangehenden Ansprüche, **dadurch gekennzeichnet, dass** der Ring (4) von mehreren Gewindelöchern (71) mit paralleler Längsachse zur zentralen Achse (X-X') oder geneigten durchquert ist, die imstande sind, Gewindestifte wie Schraubenstifte aufzunehmen, zwecks Demontage der Unterbaugruppe, gebildet aus dem Ring (4) und der Schale (3), in Bezug zum Becher (2), wobei diese Demontage durch eine Extraktion durchgeführt wird, die aus dem Druck, ausgeübt von dem Ende dieser Gewindestifte, die in dem Ring (4) ruhen, auf den Becher (2), wenn sie einer Rotation ausgesetzt sind.

7. Acetabulumprothese nach Anspruch 5, **dadurch gekennzeichnet, dass** die Gewindelöcher (71) in der äußeren ringförmigen Basis (7) ausgebildet sind.

## Claims

1. An articular prosthetic acetabulum, notably for the hip or shoulder joint, including:
- a cup (2) in a rigid and biocompatible material, having an approximately hemispherical general shape, axisymmetrical around a so-called central axis (X-X') and having a convex external face (20) adapted for being inserted and attached in the cotyloidal cavity of the bone;
- a shell (3) in an implantable material, notably in metal, ceramic or in a biocompatible composite material, able to be accommodated inside said cup (2), this shell (3) having a substantially hemispherical internal cavity (33), intended to receive the head of an articular prosthesis, and having a mouth, the external wall (31) of which has a frusto-conical shape, with low tapering, being flared outwards;
- a ring (4) able to be removably attached at the mouth of said cup (2) and having an also frusto-conical internal wall (41), the shape of which mates that of the external wall (31) of the mouth of the shell (3) so that it is possible to fit said shell (3) therein by ensuring an assembling of the Morse taper type between the ring (4) and the shell (3),
said cup (2) having an internal cavity (5) bordered by a mouth (6), while said ring (4) has an external wall portion (40), the surface of which has a shape mating that of the surface of said mouth (6), so as to be able to be axially inserted inside said mouth (6) and to be able to be disassembled relatively to said cup (2),
**characterized by** the fact that said ring (4) and said cup (2) are secured together by screwing means, said ring (4) having an external ring-shaped base (7), able to bear against the edge (21) of the cup (2) when the ring (4) is attached to the latter, this external ring-shaped base (7) being pierced with several orifices (70) for letting through fastening screws (9) intended to be screwed into tapped holes (8), with a longitudinal axis parallel to said central axis X-X', made in the wall of the cup (2), these fastening screws (9) and these tapped holes (8) being said securing means by screwing, and by the fact that the ring (4) and said cup (2) may be disassembled by unscrewing said fastening screws (9).

2. The prosthetic acetabulum according to claim 1, **characterized by** the fact that said cup (2) has an internal cavity (5) which comprises a concave bottom area (61, 62) bordered by a cylindrical mouth (6), while said ring (4) has an external wall portion (40) also cylindrical and of the same diameter, so that it may be axially inserted and practically without any play into the inside of said mouth (6).

3. The prosthetic acetabulum according to claim 2, **characterized by** the fact that the mouth (6) of the cup (2) has a stepped shape, consisting of, from the inside to the outside of the cup, two portions (6a; 6b) of different diameters, the one with the larger diameter (6b) being located on the side of the opening of the cup, both of these portions being connected through a planar ring-shaped area (50') perpendicular to said central axis X-X', and by the fact that said cylindrical external wall portion (40) of the ring (4) has a complementary stepped shape, the step of which forms a shoulder (45) able to bear against said planar ring-shaped area (50') when the ring (4) is attached to the cup (2).

4. The prosthetic acetabulum according to claim 1, **characterized by** the fact that said cup (2) has an internal cavity (5) which comprises a concave bottom area (61, 62) bordered by a cylindrical mouth (6), for which at least one portion of the internal wall has a frusto-conical shape, with large tapering, being flared outwards, while said ring (4) has an external wall portion (40) of a mating shape, so that it may be axially inserted and practically without any play into the inside of said mouth (6) and to be able to be disassembled relatively to said cup (2).

5. The prosthetic acetabulum according to claim 4, **characterized in that** the frusto-conical shape with large tapering corresponds to an apex half-angle of more than 12°.

6. The prosthetic acetabulum according to any of the preceding claims, **characterized by** the fact that said ring (4) is crossed by several tapped holes (71) with a longitudinal axis parallel to said central axis (X-X') or tilted, able to receive threaded shanks, such as screw shanks, in view to disassembling of the sub-assembly consisting of the ring (4) and of the shell (3), relatively to said cup (2), this disassembling being carried out by an extraction resulting from the pressure exerted by the end of these threaded shanks accommodated in the ring (4) against the cup (2) when they are subject to rotation.

7. The prosthetic acetabulum according to claim 5, **characterized by** the fact that said tapped holes (71) are made in said external ring-shaped base (7).
